Europäisches Patentamt

European Patent Office

Office européen des brevets

(11) Numéro de publication: **0 169 114**
**B1**

# (12) FASCICULE DE BREVET EUROPEEN

(45) Date de publication du fascicule du brevet: **16.01.91**

(21) Numéro de dépôt: **85401207.7**

(22) Date de dépôt: **18.06.85**

(51) Int. Cl.⁵: **C 12 N 15/00**, C 12 P 21/02, C 12 Q 1/37, A 61 K 37/64

(54) Dérivés de l'alpha 1-antitrypsine humaine et procédé pour leur préparation.

(30) Priorité: **19.06.84 FR 8409592**
**15.05.85 FR 8507393**

(43) Date de publication de la demande:
**22.01.86 Bulletin 86/04**

(45) Mention de la délivrance du brevet:
**16.01.91 Bulletin 91/03**

(84) Etats contractants désignés:
**AT BE CH DE FR GB IT LI LU NL SE**

(56) Documents cités:
EP-A- 164 719
EP-A-0 103 409
EP-A-0 155 188
BE-A- 895 961
NATURE, vol. 298, no. 5872, 22.07.1982, pp. 329-334; Chesham, Bucks, GB; R.W. CARRELL et al.:"Structure and variation of human alpha1-antitrypsin"
BIOLOGICAL ABSTRACTS, vol. 77, no.4, 1984,p. 2704, ref.no. 24663; Philadelphia, US; M.C. OWEN et al.:"Mutation of antitrypsin to antithrombin"

(73) Titulaire: **TRANSGENE S.A.**
**16 rue Henri Regnault**
**F-92400 Courbevoie (FR)**

(72) Inventeur: **Courtney, Michael**
**24 Grande Rue Kriegsheim**
**F-67170 Brumath (FR)**
Inventeur: **Jallat, Sophie**
**8 Quai Mathis**
**F-67000 Strasbourg (FR)**
Inventeur: **Tessier, Luc-Henri**
**20, rue du Vieux Marché aux Grains**
**F-67000 Strasbourg (FR)**
Inventeur: **Lecocq, Jean-Pierre**
**6, rue du Champ du Feu**
**F-67460 Reichstett (FR)**

(74) Mandataire: **Warcoin, Jacques et al**
**Cabinet Régimbeau 26, avenue Kléber**
**F-75116 Paris (FR)**

(56) References cited:
SCIENCE, vol. 221, no. 4605, 01.07.1983, pp. 59-61, SUMIKO INNOYE et al.: "Requirement for signal peptide cleavage of Escherichia coli prolipoprotein"

Courier Press, Leamington Spa, England.

# EP 0 208 831 B1

(56) References cited:

NATURE, vol. 313, no. 5998, 10.01.1985, pp. 149-151, London, GB, M. COURTNEY et al.:"Synthesis in E. coli of alpha1-antitrypsin variants of therpeutic potential for emphysema and thrombosis"

JOURNAL OF CELLULAR BIOCHEMISTRY, supplément, vol.0, no.9, 1985, partie B, p.94, réf.no. 0638, US; S. JALLAT et al.:"Altered inhibition properties of alpha1-antitrypsin variants constructed by site-directed mutagenesis"

# EP 0 169 114 B1

**Description**

La présente invention concerne des analogues de l'$\alpha_1$-antitrypsine humaine ainsi que des procédés pour leur préparation et des compositions pharmaceutiques contenant ces composés.

Comme cela a été décrit dans la demande de brevet français au nom de la Demanderesse n° 83 00909 et dans le brevet européen correspondant n° 84 400126 il est possible de préparer l'$\alpha_1$-antitrypsine humaine par des techniques de génie génétique.

Cette demande de brevet développe très largement les applications possibles de ce composé.

Une étude plus fine de l'activité de l'$\alpha_1$-antitrypsine humaine a permis de mettre en évidence l'intérêt que pouvaient présenter des analogues de ce produit.

Le rôle physiologique le plus important de l'$\alpha_1$-antitrypsine est l'inhibition de l'élastase neutrophile dans la partie inférieure du tractus respiratoire.

Dans les poumons sains, l'$\alpha_1$-antitrypsine et l'élastase agissent de façon équilibrée pour créer une aire de liquéfaction localisée autour des corps étrangers avant leur éjection.

Un défaut dans cet équilibre en faveur de l'élastase conduit à une action de la protéase non contrôlée et à des dommages dans les tissus très importants. Ceci conduit sur le plan clinique à un emphysème.

Un tel déséquilibre apparaît dans le cas de déficience héréditaire en l'$\alpha_1$-antitrypsine.

La cause la plus fréquente d'emphysème non héréditaire est la fumée de la cigarette. Dans ce cas il y a une interaction de divers facteurs qui conduisent à un déséquilibre protéase alvéolaire/anti-protéase. L'activité de l'$\alpha_1$-antitrypsine est diminuée par oxydation, soit directement par la fumèe de la cigarette elle-même, ou par les radicaux oxygène qui sont libérés par les macrophages attirés vers le poumon par la présence de particules de fumée polluantes. En outre ces macrophages (qui eux-mêmes produisent une élastase) sécrètent un facteur chémotactique qui conduit à un relarguage d'élastase neutrophile dans le site.

Il est également probable que l'$\alpha_1$-antitrypsine devienne sensible à une inactivation irréversible par clivage protéolytique. Ainsi, dans les poumons des gros fumeurs il existe une charge d'élastase qui peut conduire à une maladie destructrice des poumons.

Si, comme cela a été décrit dans la demande de brevet français précédente, il est possible d'utiliser l'$\alpha_1$-antitrypsine (ci-après $\alpha_1$-AT) bactérienne pour traiter les déséquilibres de tous ordres mentionnés précédemment, il semble néanmoins qu'il serait intéressant de disposer d'un analogue d'$\alpha_1$-AT présentant une stabilité améliorée in vivo.

C'est pourquoi la présente invention propose notamment des analogues de l'$\alpha_1$-AT présentant une stabilité in vivo améliorée, en particulier une résistance accrue à l'oxydation.

De nombreuses études ont démontré que l'inactivation de l'$\alpha_1$-AT est probablement due à l'oxydation du résidu méthionine à la position 358 que se trouve situé sur le site de fixation de l'élastase.

La présente invention concerne donc la préparation dans les bactéries, notamment dans *Escherichia coli,* d'une $\alpha_1$-AT humaine qui contient, à la place de l'amino-acide méthionine en position 358, un amino-acide non oxydable tel que la valine, ainsi que les analogues de l'$\alpha_1$-AT correspondants.

Les études effectuées sur les fixations de l'élastase à divers substrats indiquent que le changement de la méthionine en valine améliore la constante d'association entre l'$\alpha_1$-AT et l'élastase et augmente l'activité de l'analogue.

En outre, l'$\alpha_1$-AT inhibe une grande quantité de sérine protéases incluant l'élastase, la trypsine, la chimotrypsine, la plasmine et la thrombine. Sa fonction la plus importante, comme cela a été indiqué précédemment, est d'être un inhibiteur de l'élastase neutrophile dans le tractus respiratoire.

Le rôle de l'$\alpha_1$-AT dans le processus de coagulation est suggéré par sa capacité à inhiber la thrombine. Cette enzyme est responsable du clivage du fibrinogène en fibrine, qui est l'étape finale de la cascade de coagulation du sang.

La thrombine déclenche également l'aggrégation plaquettaire et catalyse l'activation du facteur V, du facteur VIII et du facteur XIII.

L'inactivation de la thrombine par l'$\alpha_1$-AT est un processus complexe. Dans un rapport molaire de 1:1, l'inactivation est faible et incomplète, mais en augmentant l'excès molaire de l'$\alpha_1$-antitrypsine, le taux d'inhibition augmente et la thrombine est complètement inhibée. Toutefois, comme aucun symptôme d'hypercoagulabilité n'est observé dans le cas d'un homozygote déficient $\alpha_1$-antitrypsine, il semble certain que l'$\alpha_1$-antitrypsine ne joue pas un rôle essentiel dans le contrôle de la coagulation sanguine.

Le taux d'activité anti-thrombinique dans le plasma humain peut être mis complètement sur le compte de l'activité de l'anti-thrombine III (AT-III) et de l'$\alpha$2-macroglobuline.

Dans une publication récente, un mutant $\alpha_1$-antitrypsine ($\alpha_1$-AT Pittsburgh) a été décrit qui contient un changement d'un seul nucléotide remplaçant le méthionine par l'arginine au niveau du site réactif position 358 de la molécule. Ce variant $\alpha_1$-AT a été responsable d'un désordre tragique conduisant à la mort d'un patient de 14 ans. Ce mutant $\alpha_1$-AT ne se conduit plus comme un inhibiteur de l'élastase mais présente une activité anti-thrombinique accrue de plusieurs centaines de fois. Cette activité est indépendante de l'action de l'héparine.

Ces observations peuvent s'expliquer par la comparaison de la séquence d'amino-acides de l'$\alpha_1$-AT et de AT-III. Ces inhibiteurs de protéase présentent 29% de structure commune indiquant qu'ils proviennent tous les deux d'une protéine commune.

3

Leur centre réactif présente une séquence similaire, à la position centrale de l'$\alpha_1$-antitrypsine on observe un résidu méthionine, qui est le site de clivage préféré pour l'élastase, par contre au même endroit AT-III présente un résidu arginine, qui est le site préféré pour la thrombine.

Ainsi, dans $\alpha_1$-AT Pittsburgh une substitution de la méthionine en arginine conduit à une altération de la spécificité de l'inhibition par rapport à l'élastase pour la transférer à la thrombine.

La présente invention concerne donc, tout d'abord, des analogues de l'$\alpha_1$-AT humaine dans lesquels, dans la protéine correspondant à tout ou partie d'une $\alpha_1$-AT, l'amino-acide en position 358 (normalement la méthionine) est remplacé par l'arginine ou un amino-acide pas ou peu oxydable in vivo, c'est-à-dire notamment la glycine, l'alanine, la valine, l'isoleucine, la leucine, la phénylalanine.

Dans la présente description, on entendra par "une $\alpha_1$-AT" l'un des divers variants naturels de cette protéine bien que leurs structures ne soient pas toutes connues.

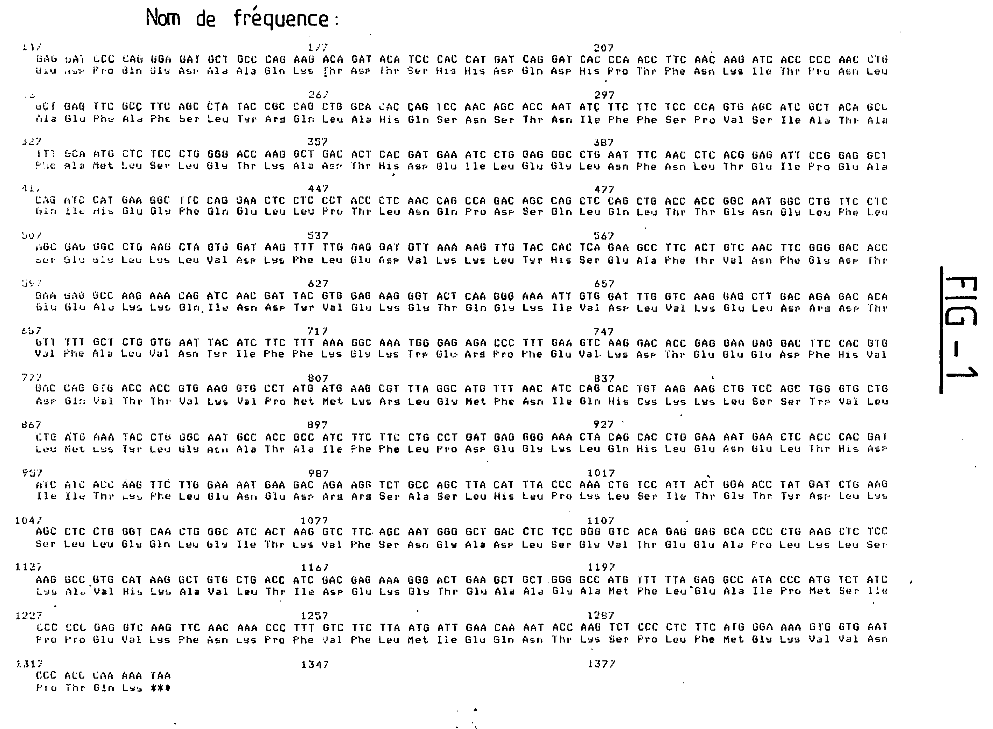

La numérotation des amino-acides est celle communément admise, le premier amino-acide étant la Glutamine dans les publications de Kurachi et coll. et Chandra et coll. (fig. 1).

De façon plus précise, l'amino-acide 358 se trouve dans la région de fixation de l'élastase et dans les composés de l'invention cette région a de préférence la structure suivante:

$$\text{ala - ile - pro - X - ser - ile}$$

$$X \text{ étant : arg, gly, ala, val, ile, leu, ou phe.}$$

Bien entendu, l'expression "une $\alpha_1$-AT" désigne également des mutants ponctuels dans lesquels une mutation a pu intervenir dans un site non réactif; cette mutation peut, en particulier, intervenir lors de la préparation des analogues par les techniques de mutations ponctuelles des génes.

Parmi les analogues selon l'invention, deux sont particulièrement intéressants:

$$[\text{Arg}^{358}]\alpha_1\text{-AT et } [\text{Val}^{358}]\alpha_1\text{-AT.}$$

En outre, des études récentes ont mis en évidence que l'$\alpha_1$-AT plasmatique normale est présente sous deux formes, une forme qui a été décrite précédemment et une autre forme qui comporte une délétion N-terminale de 5 amino-acides.

C'est pourquoi la présente invention concerne également des dérivés de l'$\alpha_1$-AT ou des analogues de l'$\alpha_1$-AT tels que décrits précédemment, caractérisés en ce que la séquence de l'$\alpha_1$-AT ou de ses analogues à été tronquée de ses 5 amino-acides N-terminaux.

D'après les études en cours, il semble que la forme tronquée provienne d'un remaniement de la molécule native dans le plasma. La forme tronquée est présente à une concentration environ 10 fois plus faible que la forme native.

Les essais effectués sur ces variants tronqués montrent qu'ils sont des inhibiteurs efficaces de l'élastase comme la protéine native.

La présente invention concerne également un procédé de préparation des analogues de l'$\alpha_1$-AT dans lequel on cultive une cellule hôte, notamment un microorganisme, comportant un vecteur d'expression de la séquence d'ADN codant pour l'$\alpha_1$-AT dans laquelle le codon correspondant à l'amino-acide 358 de la protéine mature code pour arg ou un amino-acide naturel non oxydable lorsqu'il est intégré dans une protéine ou bien dans laquelle la séquence d'ADN codant pour l'$\alpha_1$-AT ou ses analogues à été délétée de 15 bases à l'extrémité 5'.

La nature du vecteur d'expression dépend évidemment du microorganisme utilisé.

Dans le cas où le microorganisme est une bactérie, notamment *E. Coli,* le vecteur sera de préférence un plasmide comportant une origine de réplication dans *E. coli,* par exemple l'origine de réplication de pBR322.

Des plasmides d'expression pour le gène de l'$\alpha_1$-AT dans les bactéries sont décrits dans le brevet européen n° 84 400126 au nom de la Demanderesse ainsi que dans le brevet belge n° 895 961.

Parmi les plasmides vecteurs bactériens, on préfère utiliser ceux comportant un promoteur du phage $\lambda$, le promoteur $P_L$, et un site de fixation des ribosomes de la protéine cII de $\lambda$: cII$\lambda$rbs ou un site synthétique tel que décrit dans le brevet européen n° 84 400126.

En outre, ces plasmides pourront comprendre tout ou partie du gène N. D'autres caractéristiques de ces plasmides sont mises en évidence dans les exemples et le brevet cité précédemment.

Dans le cas où le microorganisme est une levure, il est possible d'utiliser comme plasmide vecteur les vecteurs décrits dans la demande de brevet européen n° 0 103 409. Lorsque la levure est une souche de *Saccharomyces cerevisiae,* le vecteur comportera, de préférence, une origine de réplication du plasmide 2$\mu$ et un promoteur de levure parmi ceux décrits dans ladite demande de brevet.

De façon générale, la structure précise du vecteur d'expression ne constitué pas une caractéristique essentielle de la présente invention.

La séquence codant pour l'analogue de l'$\alpha_1$-AT peut être préparée par des techniques connues à partir de la séquence de l'$\alpha_1$-AT naturelle d'un clone.

Il est possible de remplacer, par restriction-ligation, la partie du gène que l'on désire modifier par un gène synthétique comportant la séquence codant pour l'analogue désiré.

Mais il est également possible de procéder par mutation ponctuelle, en particulier lorsque le codon de l'analogue ne diffère que d'un nucléotide par rapport au codon de la méthionine: ATG, c'est par exemple le cas de la val: GTG, de l'arg: AGG ou de la leucine, de l'isoleucine ou de la phénylalanine.

Cette technique de mutation ponctuelle sera décrite en détail dans les exemples.

Des clones de l'$\alpha_1$-AT, ainsi que leurs préparations, sont connus et décrits dans les publications de Kurachi et coll. et Chandra et coll., ainsi que dans les divers brevets et demandes de brevet mentionnés précédemment.

Les techniques mises en oeuvre dans ces procédés sont connues dans leur principe et/ou décrites dans les documents cités.

La transformation des souches par les vecteurs ainsi que les conditions de culture des transformants afin d'obtenir les analogues de l'$\alpha_1$-AT sont également connues et sont fonction des microorganismes mis en oeuvre.

Les analogues d'$\alpha_1$-AT tronqués ou non selon l'invention peuvent être utilisés à titre de médicaments, notamment en remplacement de l'$\alpha_1$-AT pour le traitement des déficiences hériditaires ou non en $\alpha_1$-AT, par exemple pour le traitement et la prévention des emphysèmes pulmonaires.

L'analogue comportant une arginine en position 358 est un anticoagulant et sera plus particulièrement utilisable comme médicament anticoagulant mais pourra également être utilisé dans les laboratoires traitant, dosant ou stockant le sang.

Par exemple, cet analogue pourra être utilisé dans le traitement et la prévention des thromboses et en micro-chirurgie pour réduire le gonflement.

L'un des avantage de cet analogue de l'$\alpha_1$-AT est, qu'à la différence de l'héparine, il semble agir directement et non par l'intermédiaire d'un composant comme AT-III; or, dans le cas de choc opératoire, on note souvent une baisse d'AT-III, dans ces conditions l'héparine ne peut jouer son rôle d'anticoagulant à la différence de l'analogue de l'$\alpha_1$-AT.

Pour les applications en laboratoires, l'analogue de l'$\alpha_1$-AT peut être utilisé par exemple dans les circuits extracorporels ou les dosages sanguins.

Les divers variants de la présente invention sont également utilisables comme réactifs biologiques, notamment pour le dosage de l'élastase neutrophile et de la thrombine.

En particulier les variants [Leu$^{358}$]$\alpha_1$-AT et (Met$^{358}$)$\alpha_1$-AT sont utilisables comme inhibiteurs de la cathepsine G.

Les dosages utilisables dépendent, bien entendu, très largement du type de trouble à traiter et de la nature exacte de l'analogue à mettre en oeuvre et devront être adaptés par des méthodes connues de l'homme de métier.

De même, la nature précise des compositions pharmaceutiques dépendra de la voie d'application envisagée et ne constitue pas une caractéristique de la présente invention.

Enfin, ces différents composés peuvent être utilisés sous forme immobilisés sur un support approprié tel que des billes ou des tubes par exemple.

Les exemples ci-après sont destinés à mettre en évidence d'autres caractéristiques et avantages de la présente invention.

Les figures ci-annexées sont les suivantes:

La figure 1 est la séquence du cADN d'un gène humain codant pour l'$\alpha_1$-AT.

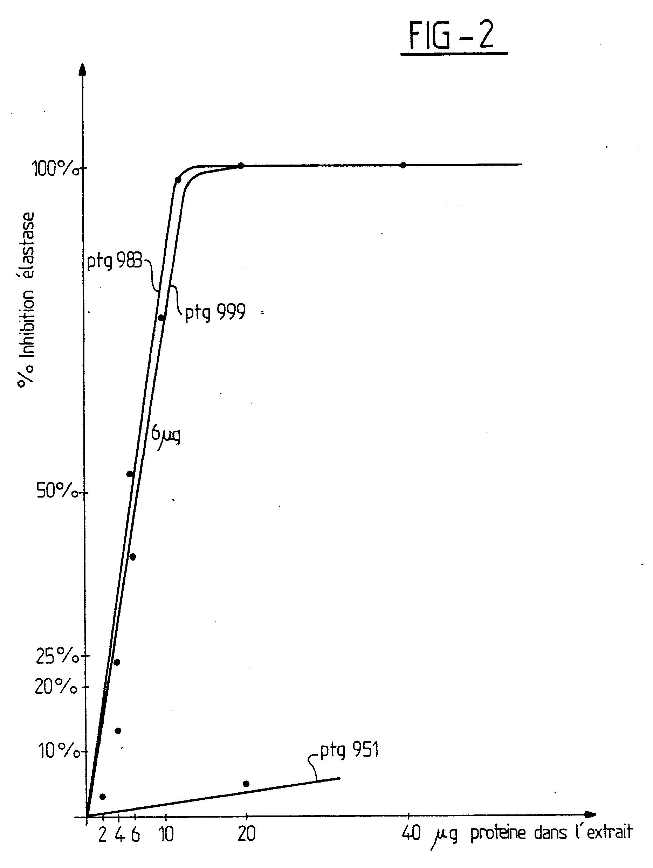

La figure 2 représente l'activité anti-élastase du produit obtenu à partir d'*E.coli/pTG999*.

La figure 3 représente l'activité anti-élastase des extraits d'*E.coli/pTG1900*.

La figure 4 représente l'activité anti-thrombinique des extraits d'*E.coli/pTG1900*.

La figure 5 représente l'activité anti-thrombinique comparée des extraits d'*E.coli/pTG1900*, de AT-III et de AT-III en présence d'héparine.

La figure 6 schématise la préparation de pTG920.

La figure 7 schématise la préparation de pTG929.

La figure 8 schématise la préparation de pTG956.

La figure 9 schématise la technique de préparation des variants par remplacement du fragment approprié d'ADN dans le gène de l'$\alpha_1$-AT.

La figure 10 représente la variation du pourcentage d'inhibition de l'élastase neutrophile humain pour différents variants.

La figure 11 représente la variation du pourcentage d'inhibition de la cathepsine G humaine pour différents variants.

La figure 12a à 12e représente les pourcentages d'inhibition d'élastase pour les variants après traitement au CNS.

La figure 13 schématise la préparation des variants tronqués.

## Exemple 1
### [Val$^{358}$]$\alpha_1$-AT

Mutagénèse directe in vitro du gène de l'$\alpha_1$-AT humain

La mutation remplaçant le méthionine par la valine nécessite le changement d'une seule base (ATG donne GTG). Une mutagénèse directe du site peut être obtenue en employant un oligonucléotide

5

synthétique (portan la version mutée de la séquence) qui peut, premièrement, s'hybrider à un modèle du gène monobrin, puis agir comme une amorce pour la synthèse du brin complémentaire grâce à l'ADN polymérase.

De cette façon, on obtient une séquence double brins, l'un des brins comportant la séquence correspondant au type sauvage, l'autre la séquence mutée.

Si un génome de M13 contenant le gène de l'$\alpha_1$-AT monobrin est utilisé comme modèle, la molécule double brins obtenue pourra être utilisée pour transformer les cellules hôtes E. coli et les phages contenant le gène mutant pourront être identifiés par screening des plaques.

Le clonage du cADN de l'$\alpha_1$-AT humain et son expression dans E. coli ont déjà été décrits, par exemple dans les brevets cités précédemment et par Courtney et coll. 1984.

Un clone produisant l'$\alpha_1$-AT, pTG983, produit l'$\alpha_1$-AT biologiquement active à un niveau correspondant à environ 1% des protéines cellulaires totales de E. coli.

Pour muter la séquence de $\alpha_1$-AT, un fragment C1al-PstI, contenant le gène, obtenu à partir de pTG983 est transféré dans le vecteur M13, M13mp701, entre les sites AccI et PstI.

L'ADN codant monobrin est alors isolé de la particule phage obtenue après infection de la cellule hôte E. coli JM103. L'oligonucléotide 5'-ATAGACACGGTATG-3' (synthétisé chimiquement) est hybridé avec l'ADN modèle en excés molaire de 100 fois par chauffage à 100°C pendant 5 minutes puis par refroidissement lent à 4°C. Cet oligonucléotide est complémentaire de la région du site actif de $\alpha_1$-AT mais contient un codon valine à la place du codon méthionine:

```
                          ala ile pro met ser ile

séquence originale : GCC ATA CCC ATG TCT ATC


                          ala ile pro val ser ile

séquence mutée     : GCC ATA CCC GTG TCT ATC
```

La synthèse du second brin est alors effectuée en incubant l'échantillon (0,5 pmol. d'ADN monobrin)pendant 2 heures à la température de la pièce avec 200 µg/ml d'ADN polymérase (fragment Klenow), 40 µ/ml de ligase ADN T4, 0,5 mM de déoxynucléotide triphosphate et 1 mM d'ATP dans un tampon (8 mM Tris-HCl, pH 7,5/8 mM de MgCl$_2$/40 mM NaCl/6 mM β-mercaptoéthanol).

L'échantillon est alors chauffé à 65°C pendant 10 minutes, une unité de ligase et 1 mM d'ATP sont alors ajoutées et incubées pendant encore 16 heures à 4°C. Des aliquotes du mélange réactionnel sont alors utilisés pour transformer le cellule compétente E. coli JM 103. Des plages de phage sont repérées sur des plaques LB et les colonies résultantes adsorbées sur un filtre de nitrocellulose et sélectionnées pour mettre en évidence la phage mutant par hybridation avec l'oligonucléotide muté marqué avec $^{32}$P en utilisant la polynucléotide kinase T4.

Dans des conditions appropriées, l'oligonucléotide forme un hybride stable seulement avec la séquence complètement homologue, c'est-à-dir mutée. Dans ce cas l'hybridation dans 3 × SSC, 1 × Denhardt, 0,5% de pyrophosphate de sodium, 0,1% SDS à 50°C, conduit à une sélection efficace.

L'ADN du phage es préparé à partir des cultures des colonies positives et utilisé pour transformer les cellules JM 103. L'ADN des plaques résultantes est alors séquencé en utilisant la procédure de terminaison des chaînes didéoxy. La séquence de nucléotide confirme qu'on a obtenu une mutation dans la position désirée du gène.

Le fragment du gène $\alpha_1$-AT muté, BGlII-PstI, est alors clivé à partir du génome de M13 et transféré dans le plasmide d'expression pTG983 après excision du fragment Bg1II/PstI. Cette manipulation conduit à un plasmide pTG999 identique à pTG983 à la seule différence que le gène codant pour l'$\alpha_1$-AT comporte une mutation au niveau du site de fixation de l'élastase.

*Expression de [Val$^{358}$]$\alpha_1$-AT dans E. coli*

L'expression du variant au niveau du site de fixation de l'élastase (pTG999) est comparée directement avec pTG983. On met en évidence que le niveau de synthése et l'activité biologique de l'$\alpha_1$-AT est comparable dans les deux clones.

La phase de culture semi-logarithmique (environ 10$^8$ cellules/ml) de pTG983, pTG999 et pTG951 (contrôle négatif correspondant à pTG983 sans le gène codant pour $\alpha_1$-AT) mis en croissance à 28°C, est induite par chauffage à 37°C pendant 5 heures. Ceci inactive le répresseur cI857 codé par l'hôte qui à basse température réprime l'expression du gène de l'$\alpha_1$-AT et bloquant la transcription à partir du promoteur PL. Après récolte, les cellules resuspendues sont broyées par sonication, les débris éliminés par centrifugation et la concentration en protéine du surnageant est estimée par une technique standard (Biorad).

Le niveau d'$\alpha_1$-AT dans chacun des extraits est déterminé par diffusion immune radiale (RID) en utilisant un kit (Calbiochem-Behring). Le diamètre de l'anneau du précipité immune est proportionnel à la concentration de l'antigène dans l'échantillon qui est calculée par comparaison avec une série de dilutions de sérums standards.

Cet essai montre que pTG983 et pTG999 produisent l'α$_1$-AT à des niveaux de 0,65 et 0,55% des protéines cellulaires totales.

Des aliquotes du surnageant obtenu après sonication sont également utilisées pour comparer l'activité anti-élastase de l'α$_1$-AT produite par pTG983 et pTG999. Ceci est effectué en testant la possibilité que présentent les extraits d'inhiber le clivage de la méthoxy-succinyl-ala-ala-pro-val-nitroanilide par l'élastase de leucocyte humain (Elastin Products Inc.). Les courbes d'inhibition de l'élastase sont représentées sur la figure 1 et démontrent clairement que pTG983 et pTG999 produisent des formes actives d'α$_1$-AT. Cette observation est importante car elle démontre que la forme variant de l'α$_1$-AT avec de la valine à la place de la méthionine au niveau du site de fixation de l'élastase est active comme la molécule naturelle.

A partir de ces courbes on a pu calculer que pTG983 et pTG999 produisent l'α$_1$-AT à des niveaux de 0,8 et 0,7% respectivement (en admettant que dans ces conditions 50 ng d'élastase sont inhibés à 50% par 50 ng d'α$_1$-AT).

<div align="center">

**Exemple 2**

**[Art$^{358}$]α$_1$-AT**
</div>

Le remplacement du codon met par arg dans le gène α$_1$-AT est effectué de la même façon que cela a été décrit précédemment. Le changement de met en arg nécessite le remplacement d'un nucléotide (ATG donne AGG):

<div align="center">

ala ile pro MET ser ile

Séquence originale : GCC ATA CCC ATG TCT ATC


ala ile pro ARG ser ile

Séquence mutée : GCC ATA CCC AGG TCT ATC
</div>

L'oligonucléotide utilisé pour effectuer cette mutagénèse est: 5'-ATAGACCTGGGTATG-3'. Cet oligonucléotide est complémentaire de la région réactive de l'α$_1$-AT mais contient un codon arginine à la place du codon méthionine. La mutagénèse directe du site est effectué sur le gène de l'α$_1$-AT cloné dans M13mp701 comme cela a été décrit précédemment. Après identification des mutants par hybridation avec l'oligonucléotide précédent, la mutation est confirmée par séquençage de l'ADN.

Le gène variant de l'α$_1$-AT sur un fragment *BG1*II-*Pst*I est alors prélevé du génome de M13 et transféré dans le vecteur d'expression pTG983 après excision du fragment *BG1*II-*Pst*I. Ceci conduit à un plasmide pTG1900 identique à pTG983 à la différence de la séquence mutée au site réactif.

*Expression de* [Arg$^{358}$]α$_1$-AT *modifiée dans E. coli*

L'α$_1$-AT produite par pTG1900 est comparée directement avec celle obtenue par pTG983. On a pu mettre en évidence que ces deux clones conduisent à la production de protéines qui réagissent de façon identique dans un test de diffusion immune radiale (RID) pour α$_1$-AT.

Le variant α$_1$-AT ne présente aucune activité anti-élastase détectable et est un inhibiteur de la thrombine très efficace.

Des extraits soumis à la sonication de cultures induites de *E. coli* TGE900 contenant pTG1900 et pTG983 sont préparés et le taux d'α$_1$-AT dans chacun des extraits est déterminé par RID. Chaque extrait contient une matière immuno-réactive et des essais indiquent que dans chaque cas le taux d'expression est de l'ordre de 1% des protéines cellulaires totales de *E. coli.*

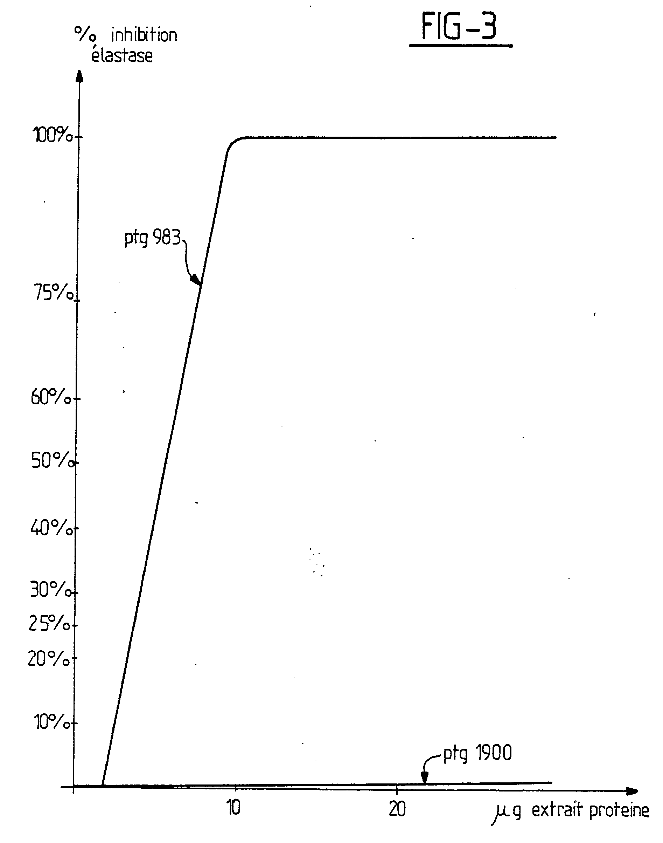

Des aliquotes de ces extraits sont également utilisées pour comparer l'activité anti-élastase et anti-thrombine de l'α$_1$-AT produite dans chacun des cas. L'activité anti-élastase est essayée en mesurant la possibilité pour les extraits d'inhiber le clivage de méthoxy-succinyl-ala-ala-pro-val-p-nitroanilide par l'élastase de leucocyte humain. Les courbes d'inhibition de l'élastase indiquent, au contraire de ce que l'on observe pour pTG983, que pTG1900 ne présente aucune activité anti-élastase (fig. 3).

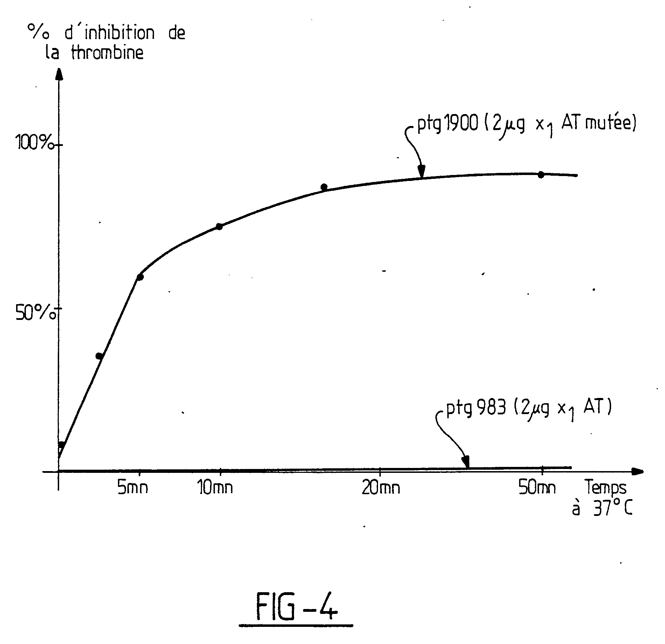

L'activité anti-thrombine est essayée en mesurant le taux d'inhibition du clivage de la chromozyme TH (tosyl-gly-pro-arg-p-nitranilide-acétate) (Boehringer) Mannheim) par la thrombine bovine. 4 μg de thrombine sont préincubés à 37°C pendant 20 minutes avec des aliquotes des extraits bactériens avant d'ajouter le substrat jusqu'à 0,1 mM. La vitesse de réaction est conduite par mesure de taux de changement de l'absorbance à 410 nm. Les résultats (fig. 4) indiquent dans ces conditions que les extraits de pTG983 ne contiennent aucune activité anti-thrombine détectable, tandis que les extraits de pTG1900 inactivent effectivement la thrombine.

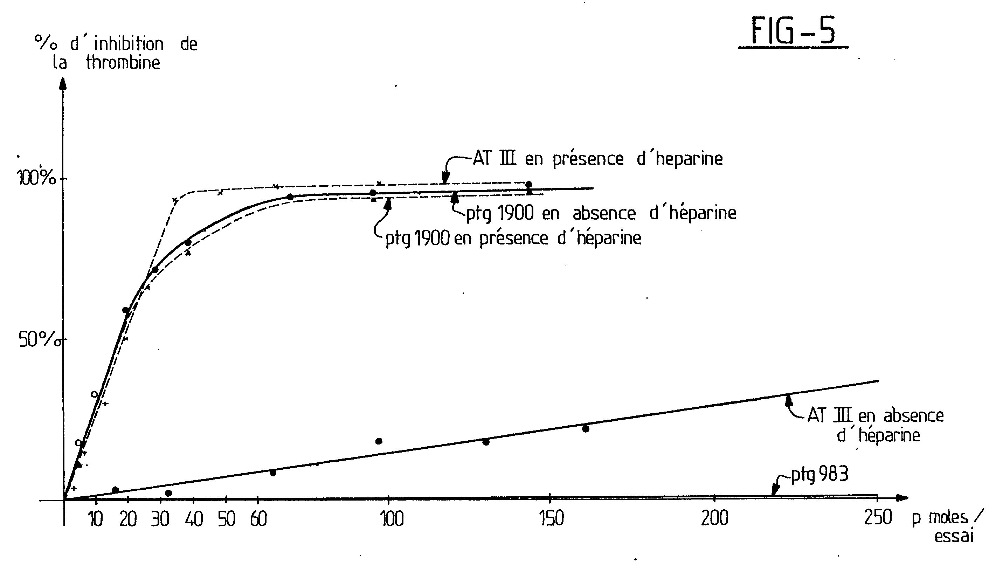

Les comparaisons de ces données avec les courbes d'inhibition d'AT-III indiquent que le variant α$_1$-AT présente une activité anti-thrombine 15 à 20 fois supérieure à celle de AT-III en l'absence d'héparine et approximativement la même activité (en moles) que AT-III en présence d'héparine (fig. 5).

Préparation de pTG983

La préparation du plasmide pTG983 est décrite dans la demande de brevet européen n° 84 400126, elle sera rappelée brièvement ci-après.

<div align="center">7</div>

La préparation du plasmide pTG983 par du plasmide pTG907 et du phage M13tg912 dont la préparation est elle-même décrite dans le brevet européen n° 094 887 au nom de la Demanderesse.

Le fragment *BamHI/SphI* de pTG907, le fragment *BGlII/HPaI* portant cllrbs et lacZ' de M13tg912 et l'adaptateur phosphorylé *HGal/SphI* ont été préhybridés dans un rapport molaire de 1:2:1 puis traités avec la ligase $T_4$. Des aliquotes sont utilisées pour transformer les cellules compétentes de la souche 6150 à 30°C.

Les cellules intéressantes sont identifiées en sélectionnant les transformants avec un fragment cllrbs/ lacZ' marqué au $P^{32}$ et la construction obtenue est confirmée par une étude de restriction enzymatique.

Afin d'avoir une première indication montrant que les différents éléments du système d'expression se conduisent comme cela est désiré, le plasmide obtenu, pTG908, est transféré dans une souche hôte N6437 qui possède à la fois cl857 et le fragment ω de la β-galactosidase complémentant le fragment α qui est codé par le plasmide.

Les transformants obtenus placés sur une boîte contenant IPTG + Xgal sont blancs à 28°C puis virent au bleu environ 30 minutes après lorsqu'on les transfère à 42°C.

Ce plasmide pTG908 a été utilisé pour exprimer le gène de l'$α_1$-AT humaine obtenu par des techniques connues e décrites précédemment.

Le clone de cADN de l'$α_1$-AT humaine utilisé, pTG603, contient un site de restriction unique *BamHI* immédiatement après le codon pour le premier amino-acide de la protéine mature. La capacité de codage pour le polypeptide mature entier, à l'exception de l'acide glutamique initial, est ainsi contenue dans un fragment *BamHI/PstI* qui a été cloné sur le vecteur d'expression pTG920; dans cette construction, la transcription est effectuée à partir du promoteur de λ à gauche, $P_L$, et la traduction est initiée à l'ATG de λcllrbs qui, accompagné par le site de fixation des ribosomes et les 39 premiers pb du gène λcll, sont fusionnés au début du gène lacZ' comme cela est indiqué.

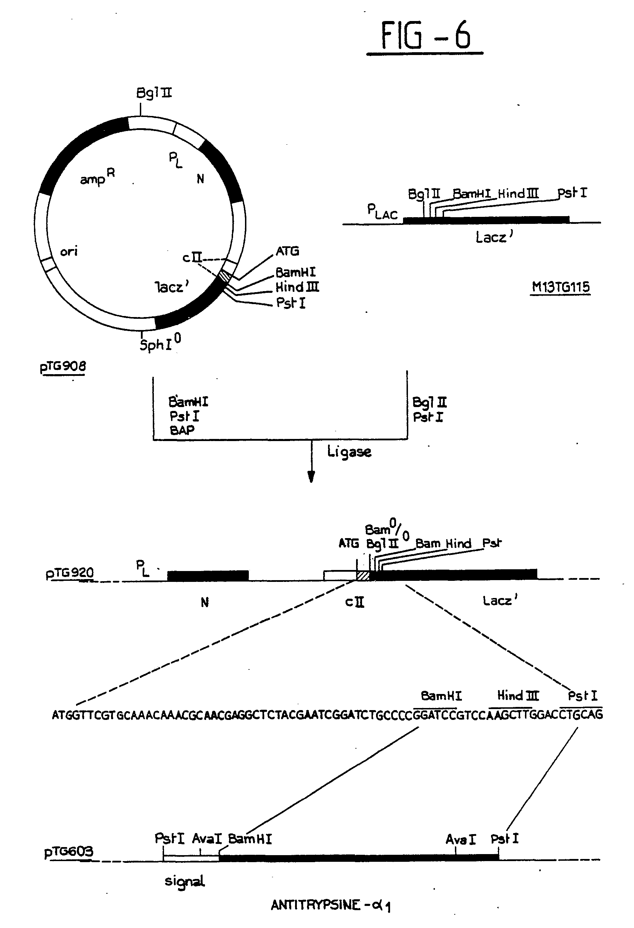

Une région de liaison comportant des sites de restriction uniques est située à la jonction de cll et de la séquence lacZ'. Le plasmide pTG920 est un dérivé de pTG908 préparé précédemment dans lequel le fragment original *BamHI/PstI* situé à 40 bp en aval de l'ATG du cll dans pTG908 es remplacé par un fragment *BGlII/PstI* de M13tg115, comme cela est décrit à la figure 6.

Grâce à ce processus, on obtient un site *BamHI* qui est placé dans la même phase de traduction que le site *BamHI* du gène de l'$α_1$-AT.

La figure 6 représente la stratégie de clonage permettant de préparer le plasmide pTG920 à partir du plasmide pTG908 et due phage M13tg115 et indication du fragment à cloner de pTG603. Les traits pleins représentent les séquences codant pour une protéine et dans le cas de pTG603 représentent la séquence codant pour le polypeptide la séquence codant pour le polypeptide mature de l'$α_1$-AT. Les régions hachurées représentent les régions codant pour les 13 amino-acides N-terminaux de cll qui seront finalement fusionnés avec l'$α_1$-AT..

Ainsi, l'insertion d'un fragment *BamHI/PstI* de pTG603 entre *BamHI* et le site *PstI* de pTG920 conduira à l'expression d'un polypeptide fusionné contenant (à partir du $NH_2$ terminal) 13 amino-acides de la protéine cll, 4 amino-acides dérivant de la séquence de l'adaptateur et le polypeptide mature de l'$α_1$-AT, excepté l'acide glutamique du $NH_2$ terminal.

pTG920, traité par *BamHI/PstI* et de la phosphatase alcaline, est lié avec pTG603, digéré avec *BamHI* et *PstI*. Les cellules TGE900 transformantes portant le fragment $α_1$-AT sont isolées après étude de restriction enzymatique. L'un de ces clones est pTG922. La préparation détaillée de ce clone est décrite dans l'article de Courtney et coll. (1984).

Le plasmide pTG922 es soumis à une restriction complète avec les enzymes de restriction *NdeI* (New England Biolabs) et *BamHI* (Bethesda Research Labs) en utilisant les conditions qui sont indiquées par le fournisseur.

On synthétise par des procédés connus des oligonucléotides adaptateurs complémentaires non phosphorylés ayant la structure suivante:

$$5'-dTATGGAG-3' \quad et \quad 5'-dGATCCTCCA-3'$$

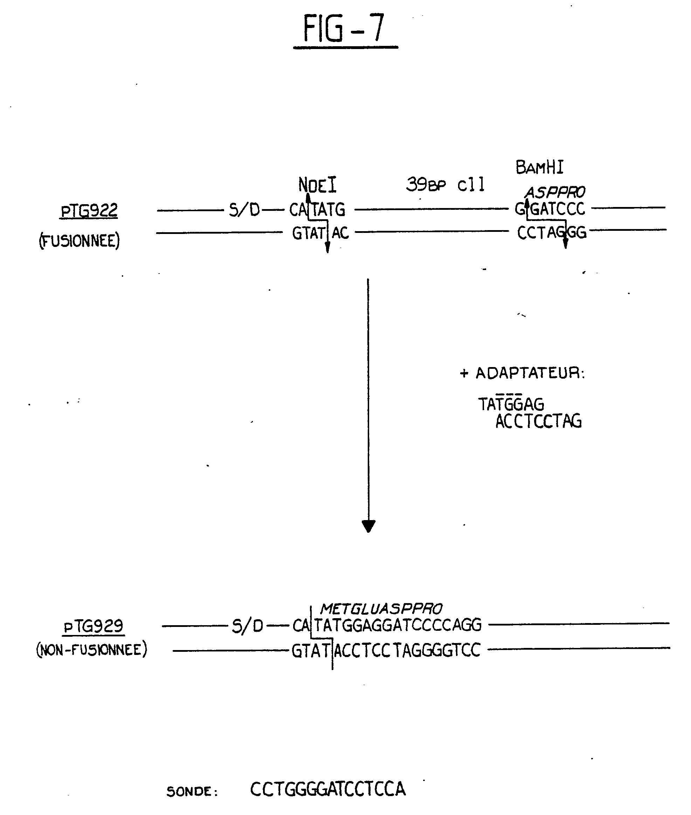

Ces oligonulcéotides sont préhybridés puis soumis à la ligation à 4°C avec le plasmide pTG922 qui à été soumis à la restriction enzymatique dans un rapport molaire de 50:1 en utilisant de l'ADN ligase dans des conditions connues (figure 7).

Le mélange de ligation est utilisé pour transformer des cellules compétentes de la souche TGE900 et les transformants obtenus sont étalés sur un milieu de culture en présence d'ampicilline.

Les colonies sont sélectionnées sur des filtres de nitrocellulose par hybridation avec une sonde marquée à la $T_4$ polynucléotide kinase 5'-dCCTGGGATCCTCCA-3'. Cette sonde complémente entièrement la construction non fusionnée que l'on désire sélectionner mais ne complémente que 7 des nucléotides du plasmide parental pTG922, ceci est insuffisant pour assurer une hybridation.

On obtient ainsi 6 candidats positifs dont l'un est appelé pTG929.

Le plasmide pTG929 ne produit que des quantités faibles d'$α_1$-AT (moins de 0,1% des protéines

cellulaires totales). De façon à augmenter le niveau d'expression, on remplace cllrbs par un site rbs synthétique:

/traduction

TCGATAACACAGGAACAGATCTATG

séquence Shine/Dalgano

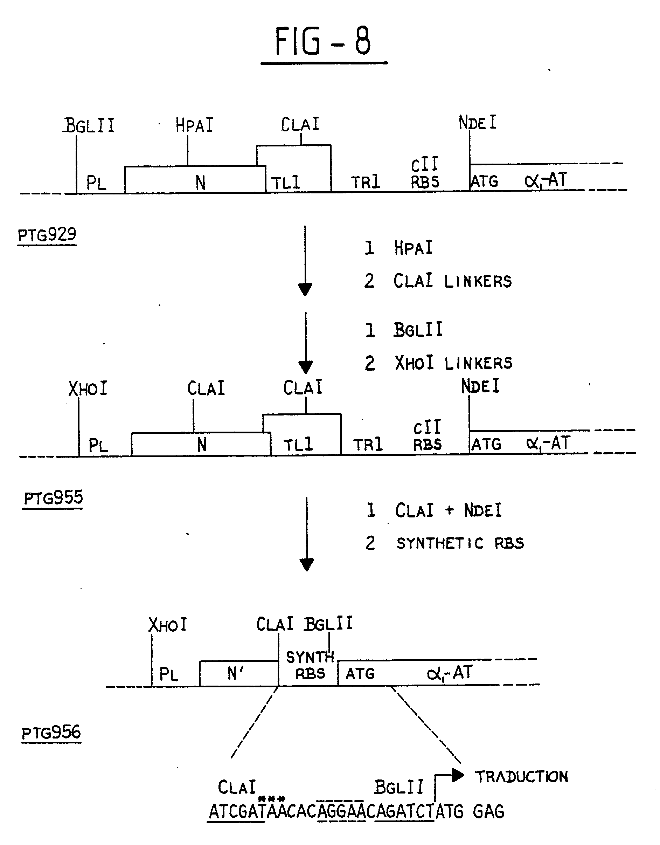

Cet échange est effectué comme cela est représenté à la figure 8.

Les sites *HPa*l et *Bg*ll de pTG929 sont remplacés en utilisant des inserts synthétiques par des sites *Cla*l et *Xho*l respectivement.

Le rbs synthétique (synth rbs) est alors inséré entre le site *Nde*l et le nouveau site *Cla*l créé dans le gène N.

Cette manipulation élimine cllrbs et conduit à un gène N tronqué immédiatement suivi de synth rbs. Un codon de terminaison de la traduction (TAA) dans synth rbs bloque la traduction de N. Le plasmide obtenu est pTG956.

On remplace dans le plasmide pTG956 la séquence originale de l'$\alpha_1$-AT par une séquence mutée:

```
                      met glu asp pro glu gly asp ala
séquence originale : ATG GAG GAT CCC CAG GGA GAT GCT


                      met glu asp pro glu gly asp ala
séquence mutée     : ATG GAA GAT CCT CAA GGC GAT GCT
                          *       *   *   *
```

Chaque changement (noté par un astérisque) est effectué en position 3 des codons et ne modifie pas l'amino-acide codé. Le gène de l'$\alpha_1$-AT a été modifié en utilisant la technique de mutagénèse dirigée sur sites en utilisant un oligonucléotide synthétique qui définit les changements de base particuliers.

Les changements de séquence choisis proviennent d'une étude statistique qui démontre une préférence pour certaines bases dans plusieurs positions au voisinage du début des gènes de *E. coli.*

Ces changements déstabiliseront également les régions correspondant à des structures secondaires possibles qui peuvent apparaître dans la séquence de mARN de l'$\alpha_1$-AT. L'oligonucléotide pour la mutagénèse est:

5'-AGCATCGCCTTGAGGATCTTCCAT-3'

Cette séquence contient 4 différences par rapport à la séquence originale et conduit aux mofications indiquées précédemment dans le gène de l'$\alpha_1$-AT.

Le plasmide obtenu, PTG983, est identique à pTG956 à l'exception des changements mentionnés précédemmment.

### Exemple 3

Preparation du variant [Gly[358]]de l'$\alpha_1$-AT

Ce variant est construit par mutagénèse directe du site, à l'aide d'un oligonucléotide synthétique, comme cela a été décrit dans l'exemple 1.

Dans ce cas-ci, l'oligonucléotide utilisé portant la mutation désirée est le suivant:

5' GATAGAACCGGGTATGGC 3'

Ceci conduit à la mutation ATG → GGT (Met→Gly)

Comme cela est décrit dans l'exemple 1, la mutagénèse est effectuée sur le gène de l'$\alpha_1$-AT sous cloné dans M13mp701. Après la mutation, le fragment BglII-PstI comprenant le gène variant est transféré dans le plasmide d'expression pTG983 décrit dans le brevet principal.

Par transformation de *E. coli* avec ce plasmide, comme décrit précédemment, on obtient l'expression du variant [Gly[358]] de l'$\alpha_1$-AT.

### Exemple 4

Préparation des variants [Lue[358]], [Ala[358]], [Ile[358]] et [Phe[358]] de l'$\alpha_1$-AT

Les différents variants mentionnés précédemment sont préparé par remplacement d'un fragment approprié d'ADN dans le gène de l'$\alpha_1$-AT.

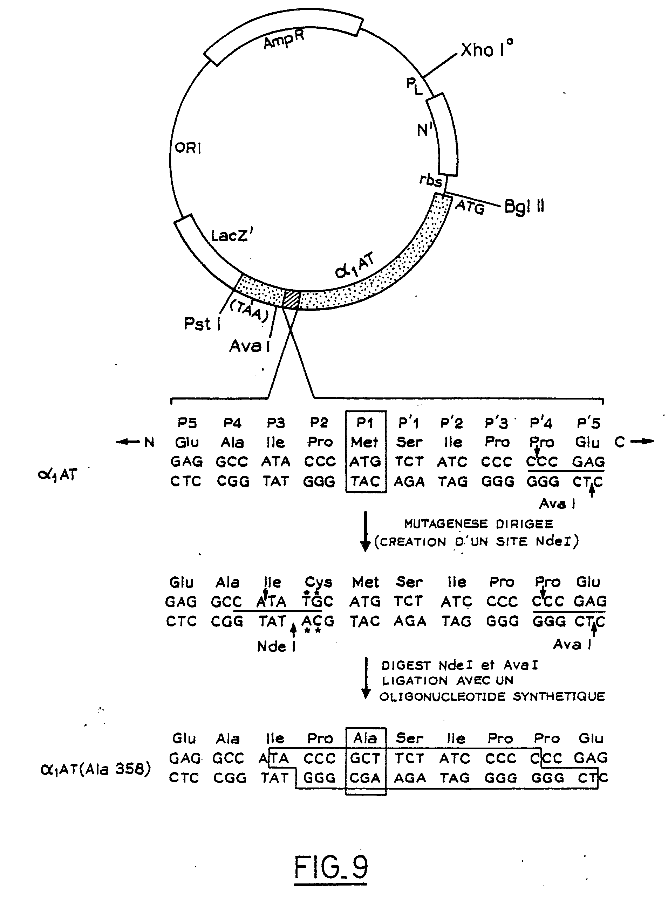

Le principe de la technique est décrit dans la figure 9 ci-annexée.

Les variants sont construits en remplaçant un petit segment d'ADN au niveau de la région codant pour l'acide aminé 358 par un fragment synthétique double-brin contenant la mutation appropriée.

9

Le petit fragment est clivé à partir du gène en utilisant les enzymes de restriction Ndel et Aval qui coupent de chaque côté de la région codant pour le site actif de 1'$\alpha_1$-AT.

Le site Aval existe déjà dans le gène d'origine, mais un nouveau site Ndel doit être créé pour la mise en oeuvre du procédé; ceci est effectué par une mutation dirigée du site par un oligonucléotide synthétique (exemple 1).

Après traitement par les enzymes de restriction, le plasmide délète de la région codant pour le site actif est séparé par électrophorèse préparative sur gel et traité avec de la phosphatase d'intestin de veau, puis cette préparation est liée, en utilisant la ligase T4, avec un petit fragment d'ADN synthétique contenant la mutation désirée ainsi que des séquences formant des extensions 5' mono-brin correspondant à celles du gène par Ndel et Aval.

Cette méthodologie conduit à des constructions identiques à celle de pTG983, à la seule différence des mutations au niveau du site actif.

Chacun des variants est produit dans *E. coli* avec des niveaux qui sont voisins de ceux obtenus pour pTG983 décrit dans le brevet principal, comme cela ressort d'essais d'immunodiffusion radiale, c'est-à-dire à des taux d'environ 1% des protéines cellulaires totales de *E. coli*.

Les extraits, soniqués et clarifiés, sont testés pour la présence de l'activité inhibitrice de l'élastase neutrophile humaine ou de la cathepsine G.

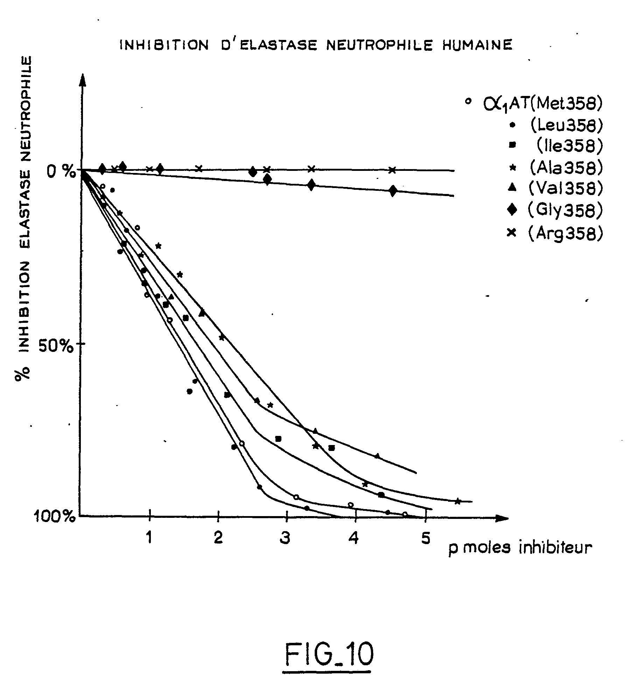

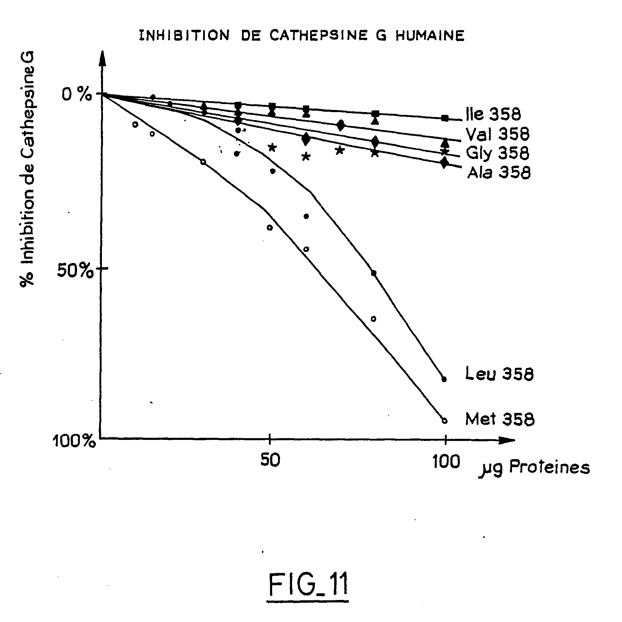

Les résultats obtenus sont rassemblés dans les figures 10 et 11 annexées, l'inhibition de l'élastase étant mesurée comme cela est décrit dans l'exemple 2 et l'activité cathepsine G étant mesurée en utilisant un substrat chromogénique, le N-succinyle-ala-ala-pro-phe-p-nitroanilide.

Les variants [Leu$^{358}$], (Ala$^{358}$] et [Ile$^{358}$] inhibent l'activité élastase comme les variants [Met$^{358}$] et [Val$^{358}$] tandis que les variants [Gly$^{358}$] et [Arg$^{358}$] ne le font pas.

De tous les variants testés, seuls les variants (Leu$^{358}$] et [Met$^{358}$] inhibent la cathepsine G.

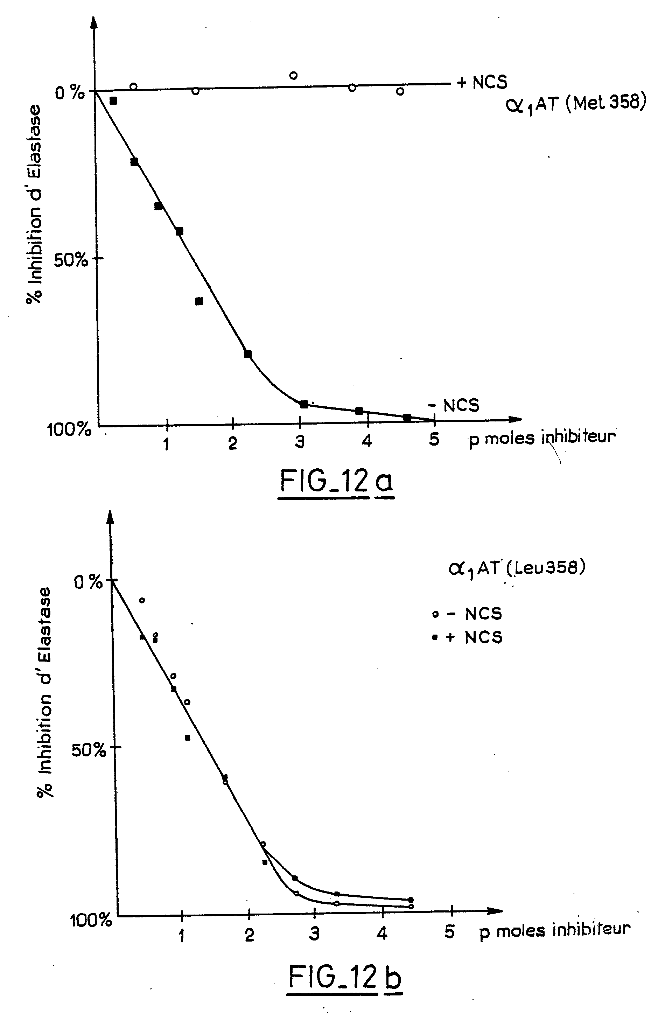

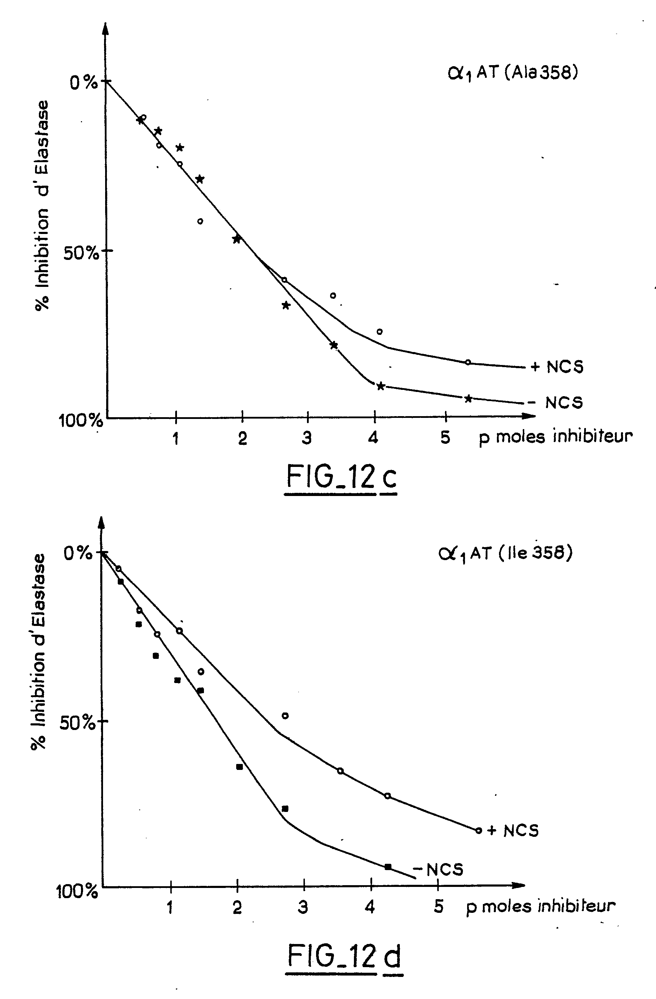

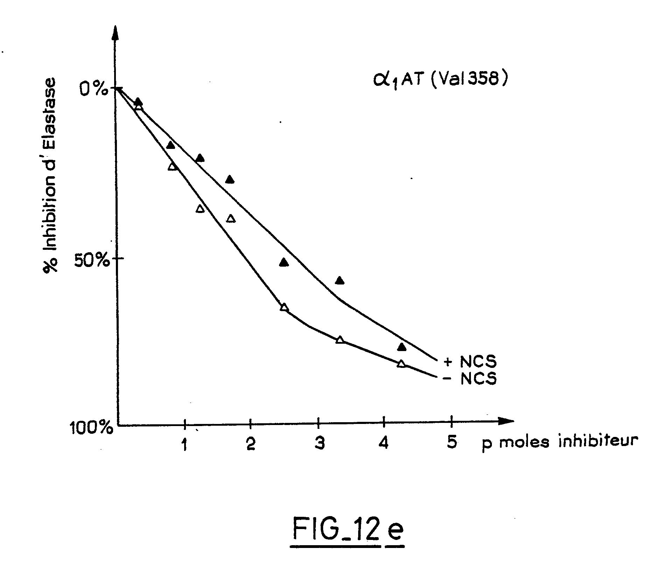

Le traitement avec 10 mM N-chlorosuccinimide (CNS) dont les résultats sont représentés à la figure 12 montre que dans des conditions où le variant [Met$^{358}$] (figure 12a) est complètement inactivé par oxydation, les variants [Leu$^{358}$] (figure 12b), [Ala$^{358}$] (figure 12c), [Ile$^{358}$] (figure 12d) et [Val$^{358}$] (figure 12e) demeurent pleinement actifs contre l'élastase neutrophile.

### Exemple 5

Production des variants tronqués de l'$\alpha_1$-AT

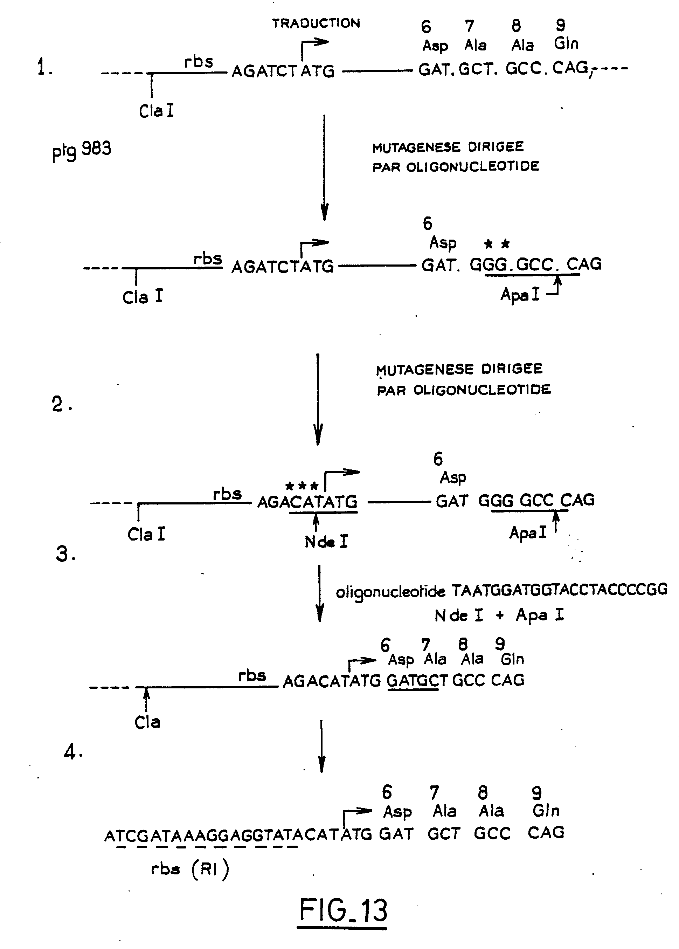

Ces variants sont préparés de la façon suivante, illustrée par la figure 13:

On part du plasmide pTG983 qui est muté à l'aide d'un oligonucléotide synthétique de façon à créer un site unique Apal autour du codon 8 en remplaçant les deux nucléotides CT du codon 7 par les nucléotides GG.

Ensuite, on effectue une nouvelle mutation du plasmide pour créer un site Ndel au niveau du codon d'initiation de la traduction.

Le plasmide résultant est traité par les enzymes Apal et Ndel de façon à déléter la partie correspondant aux amino-acides que l'on souhaite tronquer.

Ce fragment délété est replacé par un oligonucléotide synthétique qui permet d'assurer la fusion du 6ème codon de l'$\alpha_1$-AT au codon d'initiation.

Afin d'améliorer le niveau d'expression des protéines tronquées, le site de fixation des ribosomes présent sur pTG983 a été remplacé par un site de fixation des ribosomes synthétiques nommé R1 qui présente la structure représentée sur la figure 5 et qui se trouve inséré entre les sites de restriction Clal et Ndel.

Par transformation de *E. coli* à l'aide du plasmide obtenu, nommé pTG1904R1T2, on obtient une $\alpha_1$-AT dépourvue des 5 premiers amino-acides de l'extrémité N-terminale; son taux d'expression est de l'ordre de 15% des protéines cellulaires totales de *E. coli*.

Cette expression est mesurée par lecture densitométrique d'un gel d'électrophorèse coloré au bleu de Coomassie.

Les surnageants soniqués et clarifiés des cellules induites contiennent l'$\alpha_1$-AT tronquée; celle-ci inhibe efficacement l'élastase neutrophile humaine.

On observe une bonne corrélation entre le dosage par immunodiffusion radiale et l'activité anti-élastase.

Compte tenu de la mise en évidence de cette activité desdits dérivés tronqués, la présente invention s'étend également aux compositions pharmaceutiques et aux réactifs biologiques les contenant, notamment aux réactifs utiles au dosage de l'élastase neutrophile et de la thrombine.

### REFERENCES

KURACHI et al., Proc. Natl. Acad. Sci. USA, *78,* 6826-6830 (1981).

CHANDRA et al., Biochem. Biophys. Res. Comm., *103,* 751-758 (1981).

COURTNEY et coll., Proc. Natl. Acad. Sci. USA, *81,* 669-73 (1984).

**Revendications**

1. Analogue de l'alpha₁-antitrypsine humaine, caractérisé en ce qu'il s'agit:

a) de la protéine correspondant à l'alpha₁-antitrypsine humaine dans laquelle:

— en position 358, l'amino acide est choisi parmi l'arginine et les amino-acides naturels peu ou pas oxydables lorsqu'ils sont intégrés dans une protéine, et

— les 5 amino-acides N-terminaux ont été tronqués,

b) du variant (Leu³⁵⁸)alpha₁-AT.

2. Analogue selon la revendication 1:

$$[\Delta 1\text{-}5][Arg^{358}]alpha_1\text{-}AT,$$
$$[\Delta 1\text{-}5][Leu^{358}]alpha_1\text{-}AT.$$

3. Analogue selon la revendication 1:

$$[Leu^{358}]alpha_1\text{-}AT.$$

4. Analogue selon l'une des revendications 1 à 3, caractérisé en ce que sa structure au voisinage de la position 358 est la suivante:

$$\overset{358}{ala - ile - pro - X - ser - ile}$$

dans laquelle X est choisi parmi: arg, gly, ala, val, ile, leu et phe.

5. Dérivé ou analogue selon l'une des revendications 1 à 4, caractérisé en ce que l'alpha₁-AT est non glycosylée.

6. Dérivé ou analogue selon l'une des revendications 1 à 5, caractérisé en ce que l'alpha₁-AT comporte, en outre, une ou plusieurs mutations ponctuelles dans un site non réactif.

7. Procédé de préparation d'un analogue selon la revendication 1, caractérisé en ce qu'on cultive une cellule hôte, notamment un microorganisme, comportant un vecteur d'expression de la séquence d'ADN codant pour l'alpha₁-antitrypsine délétée des séquences codant pour 5 premiers amino-acides dans laquelle le codon correspondant à l'amino-acide 358 de la protéine mature code pour arg ou un amino-acide naturel non oxydable lorsqu'il est intégré dans une protéine.

8. Procédé selon la revendication 7, caractérisé en ce que le codon correspondant à l'amino-acide 358 est choisi parmi: GTC (val), AGG (arg).

9. Procédé de préparation d'un dérivé selon la revendication 7, caractérisé en ce qu'on cultive une cellule hôte, notamment un microorganisme, comportant un vecteur d'expression de la séquence d'ADN codant pour alpha₁-AT ou un analogue de l'alpha₁-AT dont les 15 bases de l'extrémité 5' ont été délétées.

10. Procédé selon l'une des revendications 7 à 9, caractérisé en ce que le microorganisme est une bactérie.

11. Procédé selon la revendication 10, caractérisé en ce que la bactérie est une souche d'*E. coli* transformée par un plasmide qui constitue le vecteur d'expression.

12. Procédé selon la revendication 11, caractérisé en ce que ledit plasmide comporte une origine de réplication dans *E. coli*, le promoteur $P_L$ assurant la promotion de la séquence d'ADN codant pour l'analogue de l'alpha₁-AT.

13. Procédé selon l'une des revendications 7 à 12, caractérisé en ce que le microorganisme est une levure.

14. Médicament caractérisé en ce qu'il comporte un analogue ou un dérivé de l'alpha₁-AT selon l'une des revendications 1 à 6.

15. Médicament destiné au traitement et à la prévention des emphysèmes pulmonaires, caractérisé en ce qu'il comporte au moins un analogue de l'alpha₁-AT selon l'une des revendications 1 à 6.

16. Réactif biologique caractérisé en ce qu'il comporte un composé selon l'une des revendications 1 à 6, utile notamment au dosage de l'élastase neutrophile et de la thrombine.

17. A titre d'agent anticathepsine G, le composé [Leu³⁵⁸]alpha₁-AT.

**Patentansprüche**

1. Analogon des Human-α₁-Antitrypsins, dadurch gekennzeichnet, daß es sich dabei handelt um

a) das Protein, das dem Human-α₁-Antitrypsin entspricht, in dem

— in der Position 358 die Aminosäure ausgewählt wird aus Arginin und den natürlichen Aminosäuren, die wenig oder nicht oxidierbar sind, wenn sie in ein Protein integriert werden, und

— die N-endständigen 5-Aminosäuren entfernt worden sind,

b) die Variante [Leu³⁵⁸]α₁-AT.

2. Analogon nach Anspruch 1 der Formel:

$$[\Delta 1\text{-}5][Arg^{358}]\alpha_1\text{-}AT,$$

$[\Delta 1\text{-}5][\text{Leu}^{358}]\alpha_1\text{-AT}.$

3. Analogon nach Anspruch 1 der Formel

$$[\text{Leu}^{358}]\alpha_1\text{-AT}.$$

4. Analogon nach einem der Ansprüche 1 bis 3, dadurch gekennzeichnet, daß seine Struktur in der Nähe der Position 358 die folgende ist:

$$\underset{\text{ala - ile - pro - X - ser - ile}}{\overset{358}{\phantom{xxxxxxxxxxxxxx}}}$$

worin X ausgewählt wird aus arg, gly, ala, val, ile, leu und phe.

5. Derivat oder Analogon nach einem der Ansprüche 1 bis 4, dadurch gekennzeichnet, daß das $\alpha_1$-AT nicht glycosyliert ist.

6. Derivat oder Analogon nach einem der Ansprüche 1 bis 5, dadurch gekennzeichnet, daß das $\alpha_1$-AT außerdem eine oder mehrere punktuelle Mutationen an einer nicht-reaktiven Stelle aufweist.

7. Verfahren zur Herstellung eines Analogons nach Anspruch 1, dadurch gekennzeichnet, daß man eine Wirtszelle, insbesondere einen Mikroorganismus, kultiviert, die (der) einen Expressionsvektor für die DNA-Sequenz trägt, die codiert für $\alpha_1$-Antitrypsin mit gelöschten Sequenzen, die für die fünf ersten Aminosäuren codieren, in dem der Codon, welcher der Aminosäure 358 des reifen Proteins entspricht, codiert für arg oder eine natürliche Aminosäure, die nicht oxidierbar ist, wenn sie in ein Protein integriert ist.

8. Verfahren nach Anspruch 7, dadurch gekennzeichnet, daß der der Aminosäure 358 entsprechende Codon ausgewählt wird aus GTC (val) und AGG (arg).

9. Verfahren zur Herstellung eines Derivats nach Anspruch 7, dadurch gekennzeichnet, daß man eine Wirtszelle, insbesondere einen Mikroorganismus, kultiviert, die (der) einen Expressionsvektor für die DNA-Sequenz trägt, die für $\alpha_1$-AT oder ein Analogon von $\alpha_1$-AT codiert, dessen 15 Basen am 5'-Ende gelöscht sind.

10. Verfahren nach einem der Ansprüche 7 bis 9, dadurch gekennzeichnet, daß es sich bei dem Mikroorganismus um ein Bacterium handelt.

11. Verfahren nach Anspruch 10, dadurch gekennzeichnet, daß es sich bei dem Bacterium um ein solches von Stamm E. coli handelt, das durch ein Plasmid, das den Expressionsvektor darstellt, transformiert worden ist.

12. Verfahren nach Anspruch 11, dadurch gekennzeichnet, daß das Plasmid einen Replikationsursprung in E. coli aufweist, wobei der Promotor $P_L$ die Promotion der DNA-Sequenz gewährleistet, die für das Analogon von $\alpha_1$-AT codiert.

13. Verfahren nach einem der Ansprüche 7 bis 12, dadurch gekennzeichnet, daß es sich bei dem Mikroorganismus um einen Hefepilz handelt.

14. Arzneimittel, dadurch gekennzeichnet, daß es ein Analogon oder ein Derivat von $\alpha_1$-AT nach einem der Ansprüche 1 bis 6 enthält.

15. Arzneimittel für die Behandlung und die Verhinderung von Lungenemphysemen, dadurch gekennzeichnet, daß es mindestens ein Analogon von $\alpha_1$-AT nach einem der Ansprüche 1 bis 6 enthält.

16. Biologisches Reagens, dadurch gekennzeichnet, daß es eine Verbindung nach einem der Ansprüche 1 bis 6 enthält, das insbesondere nützlich ist bei der Dosierung von neutrophiler Elastase und Thrombin.

17. Verbindung $[\text{Leu}^{358}]\alpha_1\text{-AT}$ als Anticathepsin G-Agens.

**Claims:**

1. Analog of human alpha$_1$-antitrypsin, characterized in that it is:
a) the protein corresponding to human alpha$_1$-antitrypsin in which:
— at the 358 position, the amino-acid is selected from among orginine and the little or not oxidizable natural amino-acids when they are integrated into a protein,
— the 5 N-terminal amino-acids have been truncated,
b) the variant $[\text{Leu}^{358}]$alpha$_1$-antitrypsin.

2. Analog according to claim 1:

$$[\Delta 1\text{-}5][\text{Arg}^{358}]\text{alpha}_1\text{-antitrypsin}$$
$$[\Delta 1\text{-}5][\text{Leu}^{358}]\text{alpha}_1\text{-antitrypsin.}$$

3. Analog according to claim 1:

$$[\text{Leu}^{358}]\text{alpha}_1\text{-antitrypsin.}$$

4. Analog according to one of claims 1 to 3, characterized in that its structure in the vicinity of the 358 position is as follows:

$$\text{358}$$
$$\text{ala - ile - pro - X - ser - ile}$$

in which X is selected from among: arg, gly, ala, val, ile, leu and phe.

5. Derivative or analog according to one of claims 1 to 4, characterized in that the $\text{alpha}_1$-antitrypsin is unglycosylated.

6. Derivative or analog according to one of claims 1 to 5, characterized in that the $\text{alpha}_1$-antitrypsin comprises, in addition, one or several point mutations in an unreactive site.

7. Process for the preparation of an analog according to claim 1, characterized in that a host cell is cultivated, particularly a micro-organism, comprising an expression vector of the DNA sequence coding for $\text{alpha}_1$-antitrypsin deleted of the sequences coding for the first 5 amino-acids in which the codon corresponding to the amino-acid 358 of the mature protein codes for arg or for a non oxidizable natural amino-acid when it is integrated into a protein.

8. Process according to claim 7, characterized in that the codon corresponding to the amino-acid 358 is selected from among: GTC (val), AGG (arg).

9. Process for the preparation of a derivative according to claim 7, characterized in that a host cell is cultivated, particularly a micro-organism, comprising an expression vector of the DNA sequence coding for $\text{alpha}_1$-antitrypsin of which the 15 bases of the 5' end have been deleted.

10. Process according to one of claims 7 to 9, characterized in that the micro-organism is a bacterium.

11. Process according to claim 10, characterized in that the bacterium is a strain of *E. Coli* transformed by a plasmid which constitutes the expression vector.

12. Process according to claim 11, characterized in that the said plasmid comprises a replication origin in *E. Coli,* the promoter $P_L$ insuring the promotion of the DNA sequence coding for the analog of $\text{alpha}_1$-antitrypsin.

13. Process according to one of claims 7 to 12, characterized in that the microorganism is a yeast.

14. Medicament characterized in that it comprises an analog or a derivative of $\text{alpha}_1$-antitrypsin according to one of claims 1 to 6.

15. Medicament for the treatment and prevention of pulmonary emphysemas, characterized in that it comprises at least one analog of $\text{alpha}_1$-antitrypsin according to one of claims 1 to 6.

16. Biological reagent characterized in that it comprises a compound according to one of claims 1 to 8, useful particularly for the assay of neutrophile elastase and of thrombin.

17. The compound [Leu$^{358}$]$\text{alpha}_1$-antitrypsin for use as an anticathepsin G agent.

# Nom de fréquence:

```
117                               177                                    207
GAG GAT CCC CAG GGA GAT GCT GCC CAG AAG ACA GAT ACA TCC CAC CAT GAT CAG GAT CAC CCA ACC TTC AAC AAG ATC ACC CCC AAC CTG
Glu Asp Pro Gln Gly Asp Ala Ala Gln Lys Thr Asp Thr Ser His His Asp Gln Asp His Pro Thr Phe Asn Lys Ile Thr Pro Asn Leu

                                  267                                    297
GCT GAG TTC GCC TTC AGC CTA TAC CGC CAG CTG GCA CAC CAG TCC AAC AGC ACC AAT ATC TTC TTC TCC CCA GTG AGC ATC GCT ACA GCC
Ala Glu Phe Ala Phe Ser Leu Tyr Arg Gln Leu Ala His Gln Ser Asn Ser Thr Asn Ile Phe Phe Ser Pro Val Ser Ile Ala Thr Ala

327                               357                                    387
TTT GCA ATG CTC TCC CTG GGG ACC AAG GCT GAC ACT CAC GAT GAA ATC CTG GAG GGC CTG AAT TTC AAC CTC ACG GAG ATT CCG GAG GCT
Phe Ala Met Leu Ser Leu Gly Thr Lys Ala Asp Thr His Asp Glu Ile Leu Glu Gly Leu Asn Phe Asn Leu Thr Glu Ile Pro Glu Ala

417                               447                                    477
CAG ATC CAT GAA GGC TTC CAG GAA CTC CTC CCT ACC CTC AAC CAG CCA GAC AGC CAG CTC CAG CTG ACC ACC GGC AAT GGC CTG TTC CTC
Gln Ile His Glu Gly Phe Gln Glu Leu Leu Pro Thr Leu Asn Gln Pro Asp Ser Gln Leu Gln Leu Thr Thr Gly Asn Gly Leu Phe Leu

507                               537                                    567
AGC GAC GGC CTG AAG CTA GTG GAT AAG TTT TTG GAG GAT GTT AAA AAG TTG TAC CAC TCA GAA GCC TTC ACT GTC AAC TTC GGG GAC ACC
Ser Asp Gly Leu Lys Leu Val Asp Lys Phe Leu Glu Asp Val Lys Lys Leu Tyr His Ser Glu Ala Phe Thr Val Asn Phe Gly Asp Thr

597                               627                                    657
GAA GAG GCC AAG AAA CAG ATC AAC GAT TAC GTG GAG AAG GGT ACT CAA GGG AAA ATT GTG GAT TTG GTC AAG GAG CTT GAC AGA GAC ACA
Glu Glu Ala Lys Lys Gln Ile Asn Asp Tyr Val Glu Lys Gly Thr Gln Gly Lys Ile Val Asp Leu Val Lys Glu Leu Asp Arg Asp Thr

657                               717                                    747
GTT TTT GCT CTG GTG AAT TAC ATC TTC TTT AAA GGC AAA TGG GAG AGA CCC TTT GAA GTC AAG GAC ACC GAG GAA GAG GAC TTC CAC GTG
Val Phe Ala Leu Val Asn Tyr Ile Phe Phe Lys Gly Lys Trp Glu Arg Pro Phe Glu Val Lys Asp Thr Glu Glu Glu Asp Phe His Val

777                               807                                    837
GAC CAG GTG ACC ACC GTG AAG GTG CCT ATG ATG AAG CGT TTA GGC ATG TTT AAC ATC CAG CAC TGT AAG AAG CTG TCC AGC TGG GTG CTG
Asp Gln Val Thr Thr Val Lys Val Pro Met Met Lys Arg Leu Gly Met Phe Asn Ile Gln His Cys Lys Lys Leu Ser Ser Trp Val Leu

867                               897                                    927
CTG ATG AAA TAC CTG GGC AAT GCC ACC GCC ATC TTC TTC CTG CCT GAT GAG GGG AAA CTA CAG CAC CTG GAA AAT GAA CTC ACC CAC GAT
Leu Met Lys Tyr Leu Gly Asn Ala Thr Ala Ile Phe Phe Leu Pro Asp Glu Gly Lys Leu Gln His Leu Glu Asn Glu Leu Thr His Asp

957                               987                                    1017
ATC ATC ACC AAG TTC TTG GAA AAT GAA GAC AGA AGG TCT GCC AGC TTA CAT TTA CCC AAA CTG TCC ATT ACT GGA ACC TAT GAT CTG AAG
Ile Ile Thr Lys Phe Leu Glu Asn Glu Asp Arg Arg Ser Ala Ser Leu His Leu Pro Lys Leu Ser Ile Thr Gly Thr Tyr Asp Leu Lys

1047                              1077                                   1107
AGC CTC CTG GGT CAA CTG GGC ATC ACT AAG GTC TTC AGC AAT GGG GCT GAC CTC TCC GGG GTC ACA GAG GAG GCA CCC CTG AAG CTC TCC
Ser Leu Leu Gly Gln Leu Gly Ile Thr Lys Val Phe Ser Asn Gly Ala Asp Leu Ser Gly Val Thr Glu Glu Ala Pro Leu Lys Leu Ser

1137                              1167                                   1197
AAG GCC GTG CAT AAG GCT GTG CTG ACC ATC GAC GAG AAA GGG ACT GAA GCT GCT GGG GCC ATG TTT TTA GAG GCC ATA CCC ATG TCT ATC
Lys Ala Val His Lys Ala Val Leu Thr Ile Asp Glu Lys Gly Thr Glu Ala Ala Gly Ala Met Phe Leu Glu Ala Ile Pro Met Ser Ile

1227                              1257                                   1287
CCC CCC GAG GTC AAG TTC AAC AAA CCC TTT GTC TTC TTA ATG ATT GAA CAA AAT ACC AAG TCT CCC CTC TTC ATG GGA AAA GTG GTG AAT
Pro Pro Glu Val Lys Phe Asn Lys Pro Phe Val Phe Leu Met Ile Glu Gln Asn Thr Lys Ser Pro Leu Phe Met Gly Lys Val Val Asn

1317                              1347                                   1377
CCC ACC CAA AAA TAA
Pro Thr Gln Lys ***
```

FIG-1

EP 0 169 114 B1

FIG -2

EP 0 169 114 B1

FIG-3

% inhibition élastase

ptg 983

ptg 1900

µg extrait proteine

FIG-4

FIG-5

% d'inhibition de la thrombine

AT III en présence d'heparine
ptg 1900 en absence d'héparine
ptg 1900 en présence d'héparine

100%

50%

AT III en absence d'héparine

ptg 983

10 20 30 40 50 60 100 150 200 250 p moles / essai

EP 0 169 114 B1

# FIG - 6

pTG908

M13TG115

BamHI
PstI
BAP

BglII
PstI

Ligase

pTG920

ATG GGTTCGTGCAAACAAACGCAACGAGGCTCTACGAATCGGATCTGCCCCGGATCCGTCCAAGCTTGGACCTGCAG

BamHI    HindIII    PstI

pTG603

signal

ANTITRYPSINE - α₁

# FIG-7

```
                                NOEI        39BP cll        BAMHI
                                                            ASPPRO
PTG922      ———— S/D —— CA|TATG ————————————— G|GATCCC ————
(FUSIONNEE) ——————————— GTAT|AC ————————————— CCTAG|GG ————
```

+ ADAPTATEUR:

TATGGAG
ACCTCCTAG

```
                                METGLUASPPRO
PTG929      ———— S/D —— CA|TATGGAGGATCCCCAGG ————————
(NON-FUSIONNEE) ——————— GTAT|ACCTCCTAGGGGTCC ————————
```

SONDE:   CCTGGGGATCCTCCA

# FIG - 8

PTG929

1  HPAI

2  CLAI LINKERS

1  BGLII

2  XHOI LINKERS

PTG955

1  CLAI + NDEI

2  SYNTHETIC RBS

PTG956

CLAI
***
ATCGATAACACAGGAACAGATCTATG GAG

BGLII

TRADUCTION

|  | P5 | P4 | P3 | P2 | P1 | P'1 | P'2 | P'3 | P'4 | P'5 |  |
| --- | --- | --- | --- | --- | --- | --- | --- | --- | --- | --- | --- |
| ← N | Glu | Ala | Ile | Pro | Met | Ser | Ile | Pro | Pro | Glu | C → |
| α₁AT | GAG | GCC | ATA | CCC | ATG | TCT | ATC | CCC | CCC | GAG |  |
|  | CTC | CGG | TAT | GGG | TAC | AGA | TAG | GGG | GGG | CTC |  |

Ava I

MUTAGENESE DIRIGEE
(CREATION D'UN SITE NdeI)

| Glu | Ala | Ile | Cys | Met | Ser | Ile | Pro | Pro | Glu |
| --- | --- | --- | --- | --- | --- | --- | --- | --- | --- |
| GAG | GCC | ATA | TGC | ATG | TCT | ATC | CCC | CCC | GAG |
| CTC | CGG | TAT | ACG | TAC | AGA | TAG | GGG | GGG | CTC |

Nde I                                          Ava I

DIGEST NdeI et AvaI
LIGATION AVEC UN
OLIGONUCLEOTIDE SYNTHETIQUE

|  | Glu | Ala | Ile | Pro | Ala | Ser | Ile | Pro | Pro | Glu |
| --- | --- | --- | --- | --- | --- | --- | --- | --- | --- | --- | --- |
| α₁AT(Ala 358) | GAG | GCC | ATA | CCC | GCT | TCT | ATC | CCC | CCC | GAG |
|  | CTC | CGG | TAT | GGG | CGA | AGA | TAG | GGG | GGG | CTC |

## FIG. 9

9

INHIBITION D'ELASTASE NEUTROPHILE HUMAINE

**FIG. 10**

INHIBITION DE CATHEPSINE G HUMAINE

FIG_11

FIG_12 a

FIG_12 b

FIG_12 c

FIG_12 d

FIG. 12 e

FIG_13